# EUROPÄISCHE PATENTSCHRIFT

(11) **EP 0 557 820 B1**
(45) Veröffentlichungstag und Bekanntmachung des Hinweises auf die Patenterteilung: **16.09.1998**
(21) Anmeldenummer: 93102222.2
(22) Anmeldetag: 12.02.1993
(51) Int. Cl.: C09B 23/16, C09B 26/02, B41M 5/38, G02F 1/35, G11B 7/24, G03G 5/06, C09B 29/036

(54) **Azinneutromethine**
Azine neutromethine compounds
Composés de type azineneutrométhine

(30) Priorität: 25.02.1992 DE 4205632
(43) Veröffentlichungstag der Anmeldung: 01.09.1993
(73) Patentinhaber: BAYER AG, 51368 Leverkusen (DE)
(72) Erfinder: Berneth, Horst, Dr., W-5090 Leverkusen 1 (DE); Hassenrück, Karin, Dr., W-4000 Düsseldorf 13 (DE)

(56) Entgegenhaltungen:
- EP-A- 0 391 631
- FR-A- 1 214 233
- PATENT ABSTRACTS OF JAPAN vol. 8, no. 23 (P-251)(1460) 31. Januar 1984 & JP-A-58 178 362 (RICOH K. K.) 19. Oktober 1983

## Beschreibung

Die vorliegende Erfindung betrifft neue Azinneutromethine, ein Verfahren zu deren Herstellung und deren Verwendung.

Es wurden nun Azinneutromethine der Formel (I) gefunden 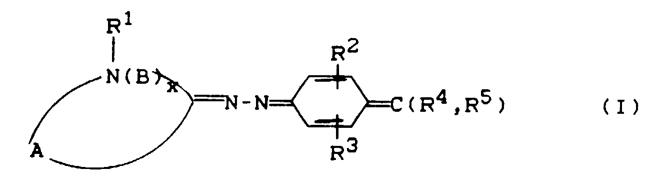 bei denen
- R¹: für C₁- bis C₂₂-Alkyl, C₄- bis C₁₀-Cycloalkyl, C₇- bis C₁₄-Aralkyl, C₆- bis C₁₀-Aryl oder einen gegebenenfalls über C₁- bis C₂-Alkylen gebundenen, 1 bis 4 Stickstoff-, Sauerstoff- und/oder Schwefelatome und 4 bis 12 C-Atome enthaltenden heterocyclischen Rest steht, wobei alle diese Reste gegebenenfalls substituiert sein können,
- R² und R³: unabhängig voneinander für Wasserstoff, Halogen, Hydroxy, Cyano, C₁- bis C₈-Alkyl, C₁- bis C₈-Alkoxy, C₄- bis C₁₀-Cycloalkoxy, C₇- bis C₁₄-Aralkyloxy oder C₁- bis C₂₀-Acylamino stehen,
- R⁴ und R⁵: unabhängig voneinander für Cyano, C₁- bis C₈-Alkoxycarbonyl, C₄- bis C₇-Cycloalkoxycarbonyl, C₆- bis C₁₀-Aryloxycarbonyl, Aminocarbonyl, Mono-C₁- bis C₈-alkylaminocarbonyl, Di-C₁- bis C₈-alkylaminocarbonyl, C₆- bis C₁₀-Arylaminocarbonyl, mit N-, S- und/oder O-haltigen 5 bis 7 Atome enthaltenden Heterocyclen substituiertes Aminocarbonyl oder durch Cyano und/oder Nitro substituiertes Phenyl stehen oder
- R⁴ und R⁵: gemeinsam mit dem dazwischenliegenden C-Atom für einen gegebenenfalls bis zu 2 Stickstoff- und/oder Sauerstoffatome enthaltenden, gegebenenfalls durch Cyano, Halogen, C₁- bis C₄-Alkyl oder C₆- bis C₁₀-Aryl substituierten 5 oder 6 Atome enthaltenden Ring stehen,
- A: den Ring, in dem es sich befindet, ergänzt zu einem gegebenenfalls benz- oder naphthanellierten, gegebenenfalls ein weiteres Stickstoff-, Sauerstoff- oder Schwefelatom enthaltenden, gegebenenfalls durch Cyano, Nitro, C₁- bis C₄-Alkyl, C₇-bis C₁₂-Aralkyl, C₆- bis C₁₀-Aryl, C₁-bis C₄-Alkoxy, gegebenenfalls über C₁-bis C₂-Alkylen gebundenen bis zu 2 N-, S- und/oder O-Atome enthaltenden 5- bis 6-gliedrigen Ring, C₁- bis C₄-Alkylthio, C₆- bis C₁₀-Arylthio, Amino, Mono- oder Di-C₁- bis C₈-alkylamino, C₄- bis C₈-Cycloalkyl-C₁- bis C₄-alkylamino, C₇- bis C₁₂-Aryl-C₁- bis C₄-alkylamino, Mono- oder Di-C₆- bis C₁₀-arylamino, Pyrrolidino, Piperidino, Morpholino oder C₁-bis C₆-Acylamino substituierten
5- bis 7-gliedrigen aromatischen oder quasiaromatischen Ring,
B für CR^{B}=CH mit R^{B}=Wasserstoff oder C₁- bis C₆-Alkyl und
x für Null oder 1 stehen.

Bevorzugte Azinneutromethine der Formel (I) sind solche, bei denen sich die Reste
- R²: in ortho-Stellung zur 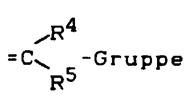 und
- _{R}3: in ortho-Stellung zur =N-N= -Gruppe und in para-Stellung zu R² befinden und
- R¹: für gegebenenfalls verzweigtes und/oder durch insgesamt bis zu drei Substituenten aus der Gruppe C₁- bis C₄-Alkoxy, Fluor, Chlor, Brom, Hydroxy, Cyano und Aminocarbonyl substituiertes C₁- bis C₂₂-Alkyl, Cyclopentyl, Cyclohexyl, Benzyl, Phenethyl, gegebenenfalls durch insgesamt bis zu 2 Substituenten aus der Gruppe C₁- bis C₄-Alkyl, C₁- bis C₄-Alkoxy, Chlor und Cyano substituiertes Phenyl oder Pyridylethyl steht,
- R²: für C₁- bis C₄-Alkyl, C₁- bis C₄-Alkoxy, Chlor, Brom, Hydroxy oder Cyano steht,
- _{R}3: für Wasserstoff, C₁- bis C₄-Alkyl, C₁- bis C₄-Alkoxy, Chlor, Brom, Hydroxy, Cyano, gegebenenfalls durch mit bis zu neun Fluoratomen substituiertes C₁- bis C₂₀-Alkanoylamino oder gegebenenfalls durch insgesamt bis zu zwei Substituenten aus der Gruppe Methyl, Methoxy und Chlor substituiertes Benzoylamino steht,
- R⁴: für Cyano, C₁- bis C₄-Alkoxycarbonyl, Cyclopentoxycarbonyl, Cyclohexoxycarbonyl, Phenoxycarbonyl, Aminocarbonyl, Mono- oder Di-C₁- bis C₄-alkylaminocarbonyl, Anilinocarbonyl oder Nitrophenyl steht,
- R⁵: für Cyano steht,
- A: den Ring, in dem es sich befindet, ergänzt zu einem Pyrazol-, Imidazol-, Triazol-, Thiazol-, Thiadiazol-, Benzimidazol-, Benzthiazol-, Pyridin-, Pyrazin-, Pyrimidin- oder Chinolinring, wobei diese Ringe gegebenenfalls durch insgesamt bis zu zwei Substituenten aus der Gruppe Chlor, Cyano, Phenyl, C₁- bis C₄-Alkyl, C₁-bis C₄-Alkoxy, C₁- bis C₄-Alkylthio, Phenylthio, Di-C₁- bis C₄-alkylamino, Cyclohexyl-C₁- bis C₄-alkylamino, Benzyl-C₁- bis C₄-alkylamino, Phenylamino, Phenyl-C₁- bis C₄-alkylamino, Pyrrolidino, Piperidino oder Morpholino substituiert sein können,
wobei die Substituenten C₁- bis C₄-Alkoxy, C₁- bis C₄-Alkylthio, Di-C₁- bis C₄-alkylamino und Cyclohexyl-C₁- bis C₄-alkylamino ihrerseits gegebenenfalls insgesamt bis zu zwei Substituenten aus der Gruppe Chlor, Methoxy, Ethoxy, Hydroxy und Cyano und die Substituenten Phenylamino und Phenyl-C₁- bis C₄-alkylamino ihrerseits gegebenenfalls insgesamt bis zu zwei Substituenten aus der Gruppe Methyl, Methoxy, Cyano und Chlor enthalten können und
B für CH=CH und
x für Null oder 1 stehen.

Besonders bevorzugte Azinneutromethine der Formel (I) sind solche, bei denen sich die Reste
- R²: in ortho-Stellung zur 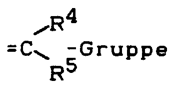 und
- _{R}3: in ortho-Stellung zur =N-N= -Gruppe und in para-Stellung zu R² befinden und
- R¹: für gegebenenfalls verzweigtes und/oder durch eine Chlor-, Cyano- oder Methoxygruppe substituiertes C₁- bis C₁₆-Alkyl oder für Benzyl steht,
- R²: für Methyl, Ethyl, Methoxy, Ethoxy oder Chlor steht,
- _{R}3: für Wasserstoff, Methyl, Ethyl, Methoxy, Ethoxy, Chlor, Cyano, Formylamino, Acetylamino, Propionylamino, Butyroylamino, Octanoylamino, Stearoylamino, Trifluoracetylamino, Nonafluorbutyroylamino, Benzoylamino, Methylbenzoylamino oder Chlorbenzoylamino steht,
- R⁴: für Cyano, Methoxycarbonyl, Ethoxycarbonyl, Amidocarbonyl oder 4-Nitrophenyl steht,
- R⁵: für Cyano steht,
- A: den Ring, in dem es sich befindet, ergänzt zu einem der Ringe (II) bis (VI) 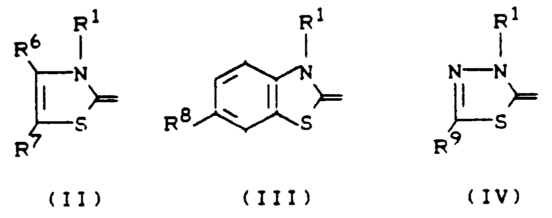 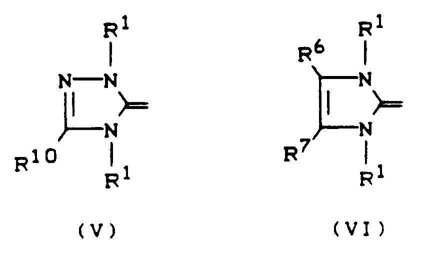 in denen
R¹ die oben angegebene Bedeutung hat,
R⁶ für Wasserstoff, Methyl, Ethyl, n-Propyl, i-Propyl, tert.-Butyl oder Phenyl und
R⁷ für Wasserstoff, Methyl, Ethyl, Chlor oder Brom oder
R⁶ und R⁷ gemeinsam für -(CH₂)₄- stehen,
R⁸ für Wasserstoff, Methyl, Methoxy oder Chlor,
R⁹ für Wasserstoff, Methyl, Ethyl, Phenyl, Methylthio, Phenylthio, Anilino, 4-Methylanilino, 4-Methoxyanilino, N-Methylanilino, Dimethylamino, Diethylamino, Dipropylamino, Dibutylamino, Dihydroxyethylamino, Dihydroxypropylamino, Hydroxyethylcyclohexylamino, Pyrrolidino, Piperidino oder Morpholino stehen,
R¹⁰ für Wasserstoff, Methyl, Ethyl, Phenyl oder Hydroxypropyl und
x für Null steht.

Bei ganz besonders bevorzugten Azinneutromethinen der Formel (I) stehen R² für Methoxy und R⁴ für Cyano und haben die sonstigen Symbole die als besonders bevorzugt angegebene Bedeutung und Stellung.

Es wurde auch ein Verfahren zur Herstellung von Azinneutromethinen der Formel (I) gefunden, das dadurch gekennzeichnet ist, daß man eine Verbindung der Formel (VII) 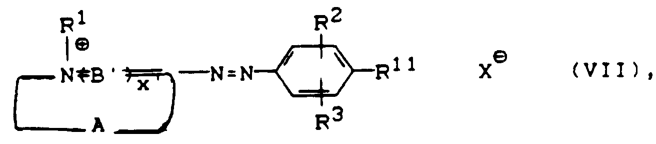 in der
- R¹, R², R³ und A: die bei der Formel (I) angegebene Bedeutung haben,
- B': für CR^{B'} -CH mit R^{B'} = Wasserstoff oder C₁ - bis C₆-Alkyl steht,
- x': für Null oder 1 steht,
- R¹¹: für Halogen, Alkoxy, Phenoxy, Monoalkylamino, Dialkylamino, N-Cycloalkyl-N-alkylamino oder N-Aralkyl-N-alkylamino steht und
- X^{⊖}: für ein Anion steht,
mit einer methylenaktiven Verbindung der Formel (VIII) 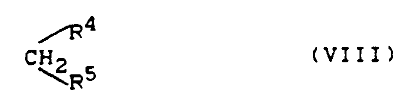 in der
R⁴ und R⁵ die bei der Formel (I) angegebene Bedeutung haben,
umsetzt.

In Formel (VII) steht R¹¹ vorzugsweise für Chlor, C₁- bis C₄-Alkoxy, Phenoxy, Mono-C₁- bis C₄-alkylamino, Di-C₁-bis C₄-alkylamino, N-C₅- bis C₇-cycloalkyl-N-C₁- bis C₄-alkylamino oder N-Phenyl-N-C₁- bis C₄-alkylamino und X^{⊖} vorzugsweise für Chlorid, Bromid, Hydrogensulfat, 1/2 Äquivalent Sulfat, Methosulfat, Dihydrogenphosphat, 1/2 Äquivalent Hydrogenphosphat, Trichlorozinkat, Tetrafluoroborat oder Perchlorat.

In den Formeln (VII) bzw. (VIII) haben R¹ bis R⁵ und A vorzugsweise und besonders bevorzugt die bei der Formel (I) als bevorzugt bzw. besonders bevorzugt angegebene Bedeutung, und B' bedeutet bevorzugt CH-CH, und x' bedeutet bevorzugt Null oder 1, besonders bevorzugt Null.

Die Verbindungen der Formel (VII) und deren Herstellung sind bekannt (siehe z.B. DE-PS 1 044 023, BE-PS 825 455, FR-PS 1 145 751, DE-OS 2 819 197, US-PS 3 051 697, FR-PS 1 462 723, FR-PS 1 378 853 und DE-OS 2 811 258) oder lassen sich auf dazu analoge Weise herstellen.

Die Verbindungen der Formel (VIII) sind bekannte Reagenzien.

Das erfindungsgemäße Herstellungsverfahren wird vorzugsweise in einem Lösungsmittel, vorzugsweise in Gegenwart einer Base und beispielsweise bei Temperaturen zwischen 0°C und dem Siedepunkt des jeweiligen Reaktionsgemisches durchgeführt.

Geeignete Lösungsmittel sind beispielsweise Wasser, Alkohole wie Methanol, Ethanol, n-Propanol, i-Propanol, Butanol, Methoxyethanol und Methoxypropanol, chlorierte Kohlenwasserstoffe wie Chloroform und 1,2-Dichlorethan, Carbonsäuren wie Essigsäure und dipolare Lösungsmittel wie Acetonitril, Dimethylformamid, N-Methylpyrrolidon, Dimethylsulfoxid und Tetramethylharnstoff, sowie beliebige Gemische dieser Lösungsmittel.

Geeignete Basen sind beispielsweise Alkali- und Erdalkalihydroxide und -oxide wie Natriumhydroxid, Kaliumhydroxid, Calciumhydroxid und Magnesiumhydroxid, Alkaliund Erdalkalicarbonate und -hydrogencarbonate wie Natriumcarbonat, Natriumhydrogencarbonat, Kaliumcarbonat und Calciumcarbonat, Alkoholate wie Natriummethylat, Natriumethylat und Kalium-tert.-butylat, sowie Amine wie Triethylamin, Triethanolamin, Triisopropanolamin und N,N-Dimethylanilin und Salze von Carbonsäuren wie Natrium- und Kaliumacetat.

Man kann, bezogen auf die jeweilige Verbindung der Formel (VII), beispielsweise eine äquimolare Menge Base oder einen Überschuß an Base einsetzen.

Bevorzugte Reaktionstemperaturen sind solche zwischen 15 und 50°C.

Es wurde auch gefunden, daß die Azinneutromethine der Formel (I) als Farbstoffe in thermischen und fotochromen Aufzeichnungsmedien, als nichtlineare optische Materialien und als Fotoleiter in der Elektrofotografie verwendet werden können. Diese Verwendungsmöglichkeiten können beispielsweise wie folgt realisiert werden:

Thermische Aufzeichnungsmaterialien können in zwei Kategorien eingeteilt werden. In einem Fall wird der Farbstoff als solcher übertragen und gibt eine farbige Markierung. Im zweiten Fall wird eine farblose Form des Farbstoffs durch Wärmeeinwirkung in eine farbige Form überführt.

Beispiele für den ersten Fall sind Farbstoffdiffusions- und Farbstoffsublimation-Transferdruck-Verfahren. Hierbei wird zunächst ein gut diffundierender oder sublimierender Farbstoff in eine Schicht eingebracht, die sich auf einem dünnen Trägermaterial, z.B. aus Polyester, befindet. Wird diese farbgebende Schicht in Kontakt mit einer Empfängerschicht gebracht, die sich ebenfalls auf einem Trägermaterial befindet, so läßt sich durch einen Thermodruckkopf, der mit elektrischen Signalen gesteuert wird, der Farbstoff von der farbgebenden Schicht auf die Empfängerschicht übertragen und bildet dort ein den elektrischen Signalen entsprechendes Muster. Ein solches Aufzeichnungsmedium wird beispielsweise in der EP-OS 384 040 beschrieben.

Die erfindungsgemäßen Azinneutromethin-Farbstoffe können mit Lösungsmitteln in eine solche farbgebende Schicht eingebracht werden. Sie zeichnen sich durch gutes Sublimierverhalten aus. Geeignete Lösungsmittel für diese Anwendung sind z.B. Ether wie Tetrahydrofuran, chlorierte Kohlenwasserstoffe wie Chloroform, 1,2-Dichlorethan und Chlorbenzol, Ester wie Essigsäureethylester und Essigsäurebutylester und dipolare Lösungsmittel wie Acetonitril, Dimethylformamid, N-Methylpyrrolidon, Dimethylsulfoxid und Tetramethylharnstoff, sowie Mischungen davon.

Die erfindungsgemäßen Azinneutromethin-Farbstoffe können gemäß dem zweiten Fall z.B. als stark gefärbte Lösung in einem Lösungsmittel, z.B. in einem der zuvor genannten Lösungsmittel, auf ein Substrat aufgetragen werden. Läßt man das Lösungsmittel verdampfen, z.B. durch Verdunstung oder im Vakuum, so entfärbt sich die Beschichtung. Sie ist anschließend farblos oder nur noch schwach gefärbt. Bei Einwirkung von Wärme entwickelt sich wieder eine intensive Färbung, die stabil ist.

Fotochrome Aufzeichnungsmaterialien können auf einem Trägermaterial aus beispielsweise Kunststoff einen Farbstoff oder ein farbloses Derivat eines Farbstoffs enthalten. Sie können auch als Langmuir-Blodgett-Filme auf einem Trägermaterial aufgebracht sein. Eine bildmäßige Belichtung dieser Beschichtung führt zu einer Farbänderung oder Farbentwicklung, die für optische Datenaufzeichnungen verwendet werden kann. Solche Materialien sind beispielsweise beschrieben in den EP-OS'en 193 931 und 391 631.

Die farblose oder nur schwach gefärbte Schicht, die erfindungsgemäße Azinneutromethine beim Auftragen ihrer Lösungen auf ein Substrat nach dem Entfernen des Lösungsmittels ergeben, färbt sich bei Einwirkung von Licht spontan intensiv und über längere Zeit stabil an. Deshalb können erfindungsgemäße Azinneutromethine als Bestandteil fotochromer Aufzeichnungsmaterialien verwendet werden.

Nicht-lineare optische Materialien besitzen eine feldstärkeabhängige dielektrische Suszeptibilität zweiter Ordnung, die eine Reihe dispersiver Effekte zur Folge haben kann. So erlaubt z.B. die Frequenzverdoppelung die Erzeugung von Licht der verglichen mit dem eingestrahlten Licht halben Wellenlänge. Der elektrooptische Effekt ermöglicht eine Änderung des Brechungsindex bei angelegtem elektrischen Feld. Die Frequenzmischung und die Frequenzteilung gestatten die kontinuierliche Abstimmung von Laserlicht. Nicht-lineare optische Materialien können aufgrund dieser Effekte z.B. als elektrooptische Schalter, zur Frequenz- und Intensitätssteuerung von Laserlicht, für die Holographie, Informationsverarbeitung und integrierte Optiken verwendet werden. Derartige Anwendungen sind z.B. beschrieben von D. Lupo et al. in Adv. Materials 3, 54 (1991) und in der DE-OS 3 743 833.

Die erfindungsgemäßen Azinneutromethine zeigen bei Belichtung mit Laserlicht eine Emission von Licht der halben Wellenlänge. Sie sind deshalb nicht-lineare optische Materialien.

In der Elektrofotografie werden Fotoleitertrommeln eingesetzt, die organische Farbstoffe und Pigmente als Fotoleiter enthalten. Hierbei wird auf ein metallisches Trägermaterial eine Schicht eines geeigneten fotoleitenden Farbstoffs oder Pigments in einem Bindemittel aus Kunststoff aufgetragen. Darüber wird eine Schicht einer ladungsleitenden Substanz in einem organischen Bindemittel aufgebracht. Die Trommel wird mittels einer Corona-Entladung auf einige hundert Volt aufgeladen. Eine bildmäßige Belichtung der geladenen Trommel läßt an den belichteten Stellen die Ladung abfließen. Die so bildmäßig geladene Trommel nimmt nun entgegengesetzt geladenes Tonerpulver auf und überträgt dieses bildmäßig auf ein Blatt Papier. Ein solches System ist beispielsweise beschrieben von D.M. Burland und L.B. Schein in Physics Today 1986, 46.

Die erfindungsgemäßen Azinneutromethine führen als Fotoleiterfarbstoffe in einem solchen elektrofotografischen Prozeß zunächst zu einer hohen Aufladung und dann zu einer guten Entladung bei Belichtung.

Erfindungsgemäße Azinneutromethine der Formel (I) sind auch geeignet zum Färben von Fasern und Geweben aus Polyester und von Kunststoffen, beispielsweise Polystyrol, Polycarbonat und Acrylnitril/Butadien/Styrol-Copolymeren.

### Beispiele

### Beispiel 1

a) 167 g 2-Amino-5-diisopropylamino-1,3,4-thiadiazol wurden in einer Mischung aus 400 ml Eisessig, 120 g 48 gew.-%iger wäßriger Schwefelsäure und 40 g 85 gew.-%iger wäßriger Phosphorsäure mit 272 g 42 gew.-%iger Nitrosylschwefelsäure bei -5°C diazotiert. Die erhaltene Lösung wurde bei 25°C zu einer Lösung von 156 g 4-Acetaminoveratrol in 400 ml Eisessig getropft. Nach 1 Stunde wurden 263 g Natriumacetat zugesetzt und der ausgefallene Farbstoff abgesaugt und getrocknet. Es ergaben sich 294 g eines roten Pulvers der Formel (IX) 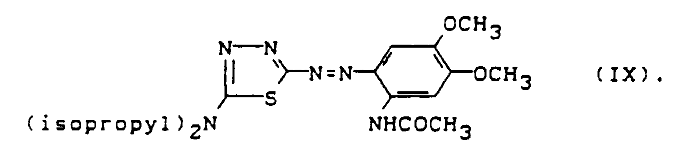
b) 20,3 g des so erhaltenen Farbstoffs wurden in 100 ml Eisessig mit 20 g Butylbromid 5 Stunden lang bei 70°C gerührt. Danach wurde mit 500 ml Wasser verdünnt, mit Aktivkohle geklärt, mit 30 g Natriumchlorid und abschließend mit 25 ml 2-molarer wäßriger Zinkchloridlösung versetzt. Der ausgefallene Feststoff wurde abgesaugt und getrocknet. Es ergaben sich 22,6 g eines violetten Pulvers der Formel (X) 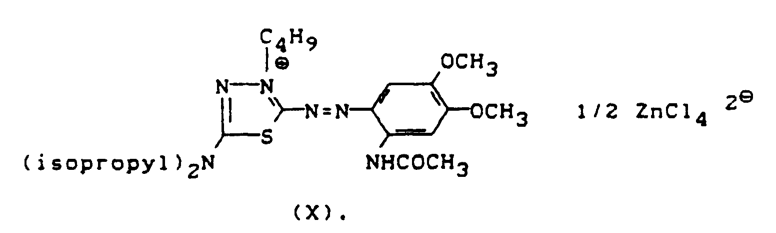
c) 11,3 g dieses Farbstoffs wurden in 160 ml Methanol zusammen mit 1,45 g Malodinitril vorgelegt. Danach wurden bei Raumtemperatur langsam 4,24 g Natriumcarbonat eingestreut. Es fiel ein dicker Niederschlag aus, der abgesaugt und mit Methanol und Wasser gründlich gewaschen wurde. Nach dem Trocknen wurden 7,2 g (73 % d. Th.) grünlich schimmernde Plättchen mit einem Schmelzpunkt von 233°C erhalten. Das Produkt entsprach der Formel (XI) 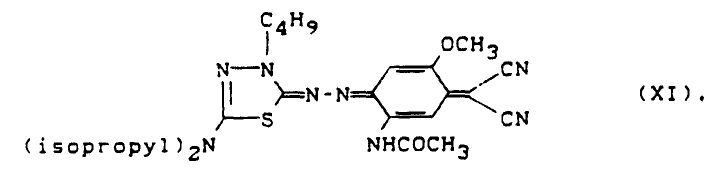 Der λₘₐₓ-Wert von (XI) in Dimethylformamid betrug 640 nm.

### Beispiel 2

4,2 g einer Verbindung der Formel (XII) 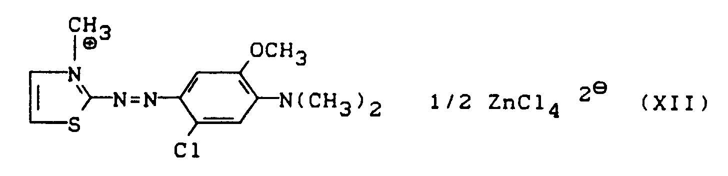 wurden in 50 ml Methanol mit 730 mg Malodinitril und 2,1 g Natriumcarbonat versetzt. Nach 2 Stunden wurde abgesaugt und mit Methanol und Wasser gründlich gewaschen. Nach dem Trocknen wurden 2,3 g (70 % d.Th.) der Verbindung der Formel (XIII) in Form violetter Plättchen erhalten

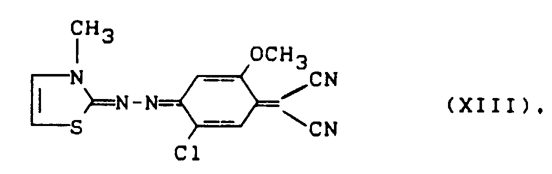

Für die Verbindung der Formel (XIII) wurden folgende λₘₐₓ-Werte bestimmt: 632 nm in Dimethylformamid und 608 nm in Chloroform. Das IR-Spektrum wies eine charakteristische Bande bei 2200 cm⁻¹ auf.

### Beispiele 3 bis 26

Analog Beispiel 1c oder 2 wurden ausgehend von den entsprechenden kationischen Farbstoffen folgende Farbstoffe der Formel (XIV) erhalten

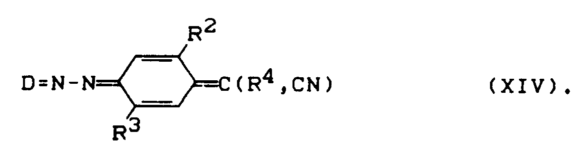

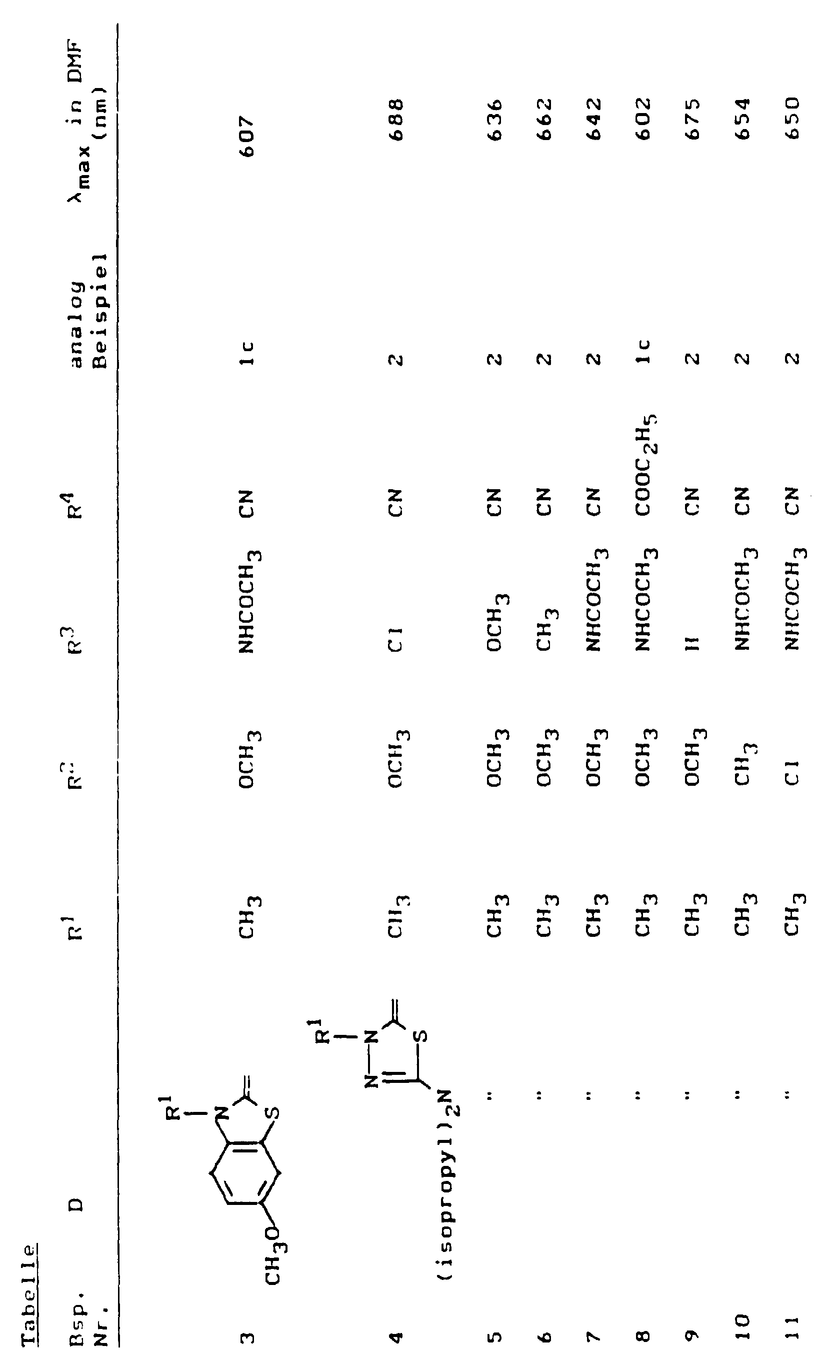

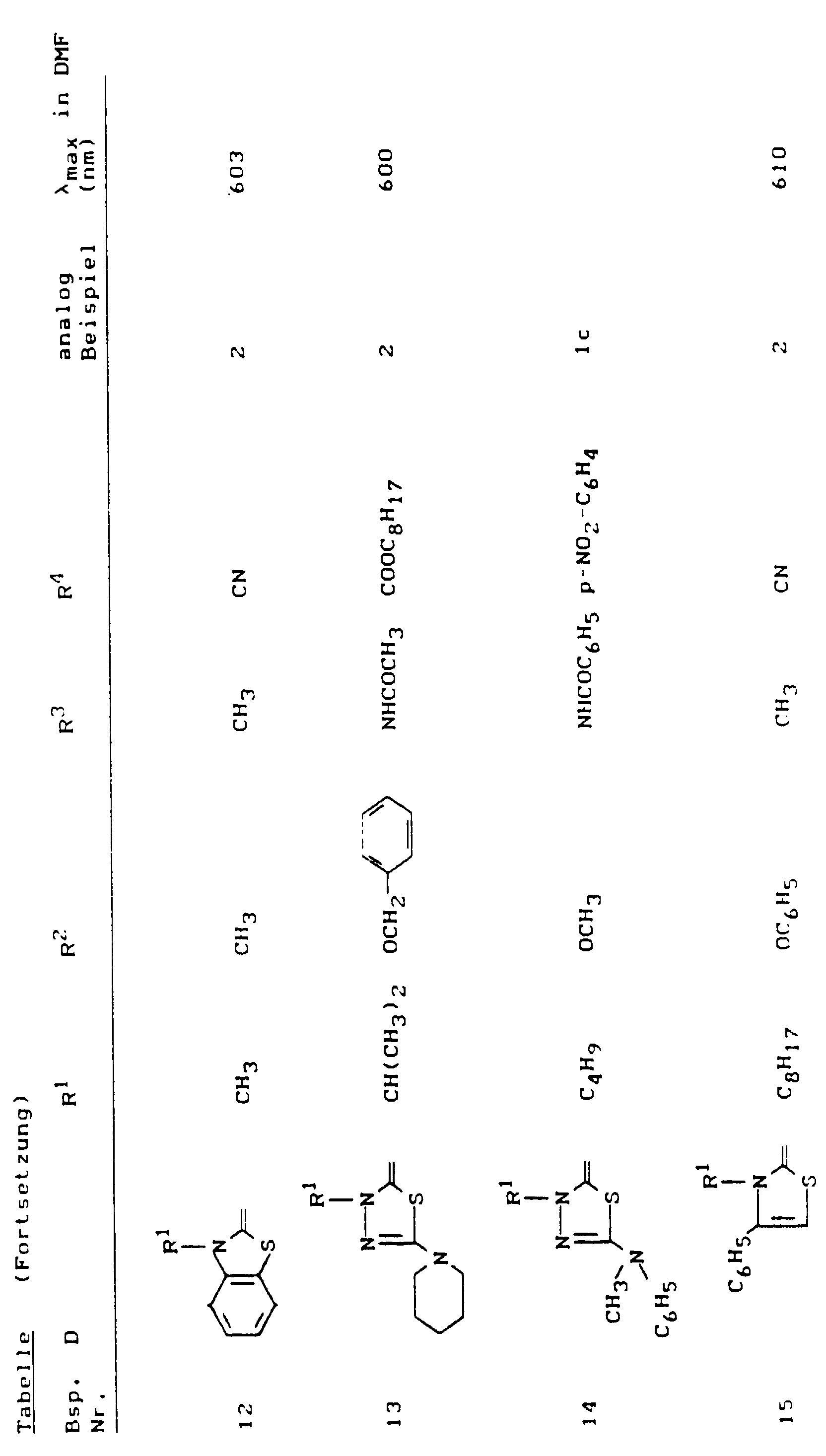

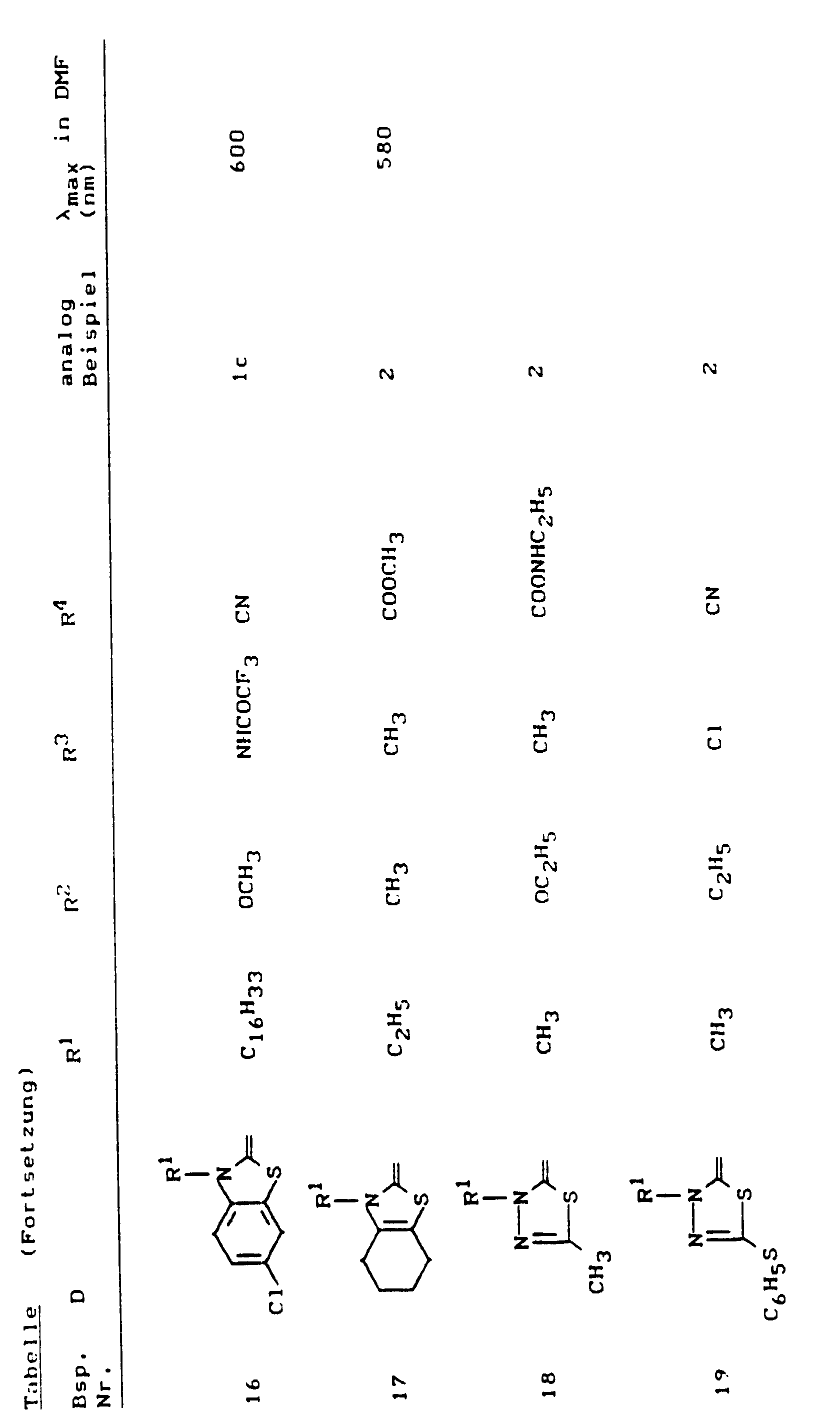

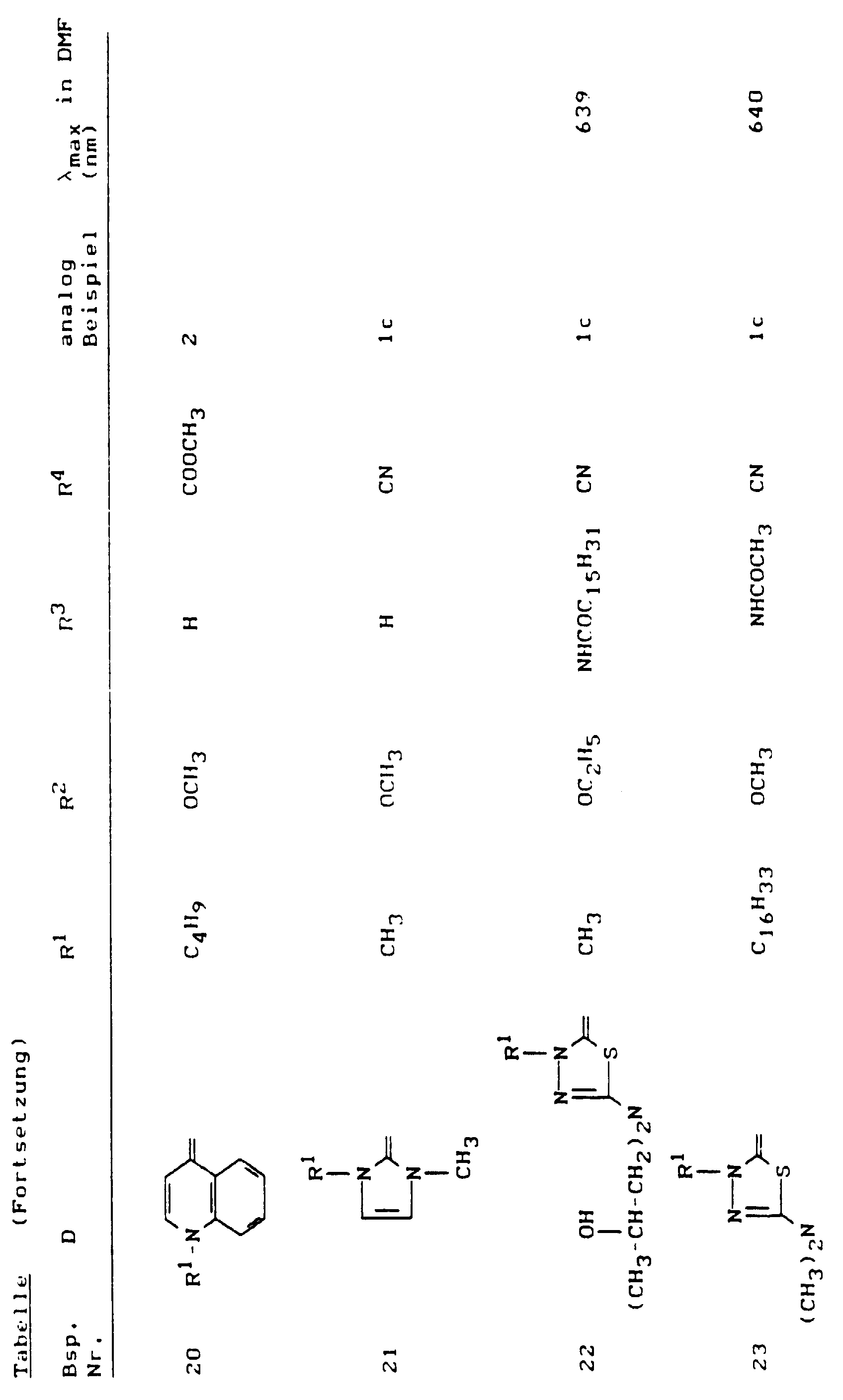

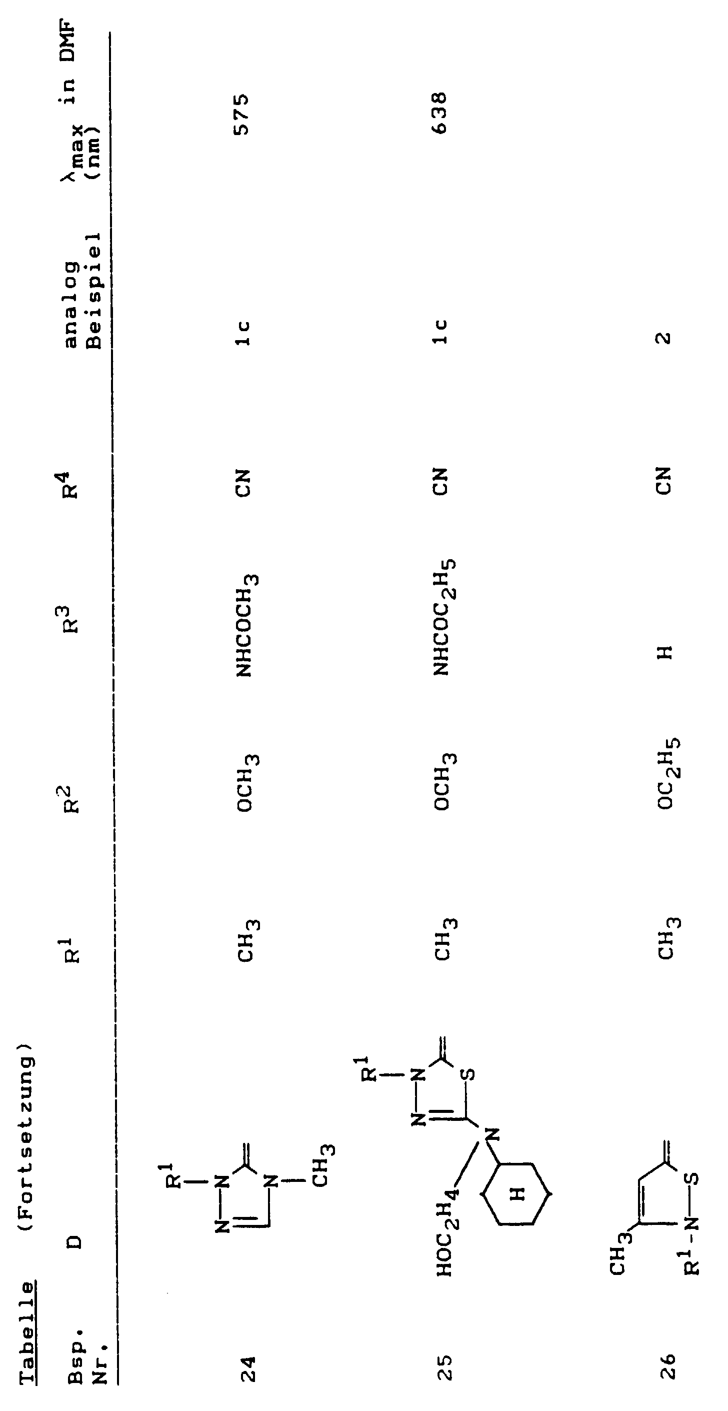

### Beispiele 27 bis 29

Analog Beispiel 1c oder 2 wurden ausgehend von den entsprechenden kationischen Farbstoffen folgende Farbstoffe der Formel (I) erhalten:

### Beispiel 27

Bei dem erhaltenen Farbstoff der Formel (I) ergänzte A den Ring, in dem es sich befindet, zu 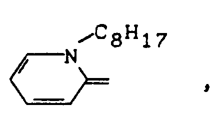 standen R² für OCH₃ und R³ für Wasserstoff und R⁴ und R⁵ für COOC₂H₅. Gearbeitet wurde analog Beispiel 2.

### Beispiel 28

Bei dem erhaltenen Farbstoff der Formel (I) ergänzte A den Ring, in dem es sich befindet, zu 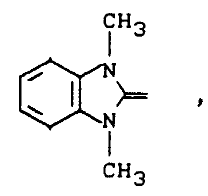 standen R² und R³ für OCH₃ und R⁴ und R⁵ gemeinsam für den Rest

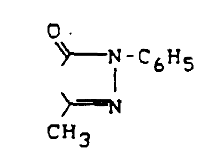

Gearbeitet wurde analog Beispiel 2.

### Beispiel 29

Bei dem erhaltenen Farbstoff der Formel (I) ergänzte A den Ring, in dem es sich befindet, zu 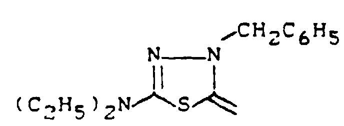 standen R² für OCH₃, R³ für NHCOCH₃ und R⁴ und R⁵ für COCH₃. Gearbeitet wurde analog Beispiel 1c.

### Beispiel 30 (Diffusionstransferdruck)

Das Farbstoffgeberelement wurde folgendermaßen hergestellt:
eine 0,5 gew.-%ige Lösung des Farbstoffs des Beispiels 1 und eine 0,5 gew.-%ige Lösung eines Binders auf Basis Styrol/Acrylnitril-Copolymerisat in Tetrahydrofuran wurden mit einer Schichtdicke von 100 µm im feuchten Zustand auf einen 5 µm dünnen Polyethylenterephthalat-Film aufgetragen, der vorher mit einer Schicht aus Vinylidenchlorid/Acrylnitril-Copolymerisat (2,5 µm Stärke im trockenen Zustand) versehen worden war. Die gebildete Schicht wurde im Vakuum getrocknet. Die Rückseite des Polyethylenterephthalat-Filmes wurde mit einer Lösung beschichtet, die 5 Gew.-% Styrol/Acrylnitril-Copolymer und 0,1 Gew.-% einer 1 gew.-%igen Lösung eines Polysiloxan/Polyether-Copolymers -Copolymers (Tegoglide® 410 der Fa. Th. Goldschmidt) enthielt. Diese Lösung wurde in einer Dicke von 100 µm aufgetragen und diente zur Verhinderung des Anklebens des Farbgeberelements an den Thermodruckkopf. Das so erhaltene Farbstoffgeberelement wurde in einem Hitachi color video printer VY-100 A zum Drucken auf im Handel erhältliches Aufzeichnungsmaterial (Hitachi VY-S 100 A paper ink set) eingesetzt.

Es wurde eine intensive cyanfarbene Aufzeichnung erhalten.

Analoge Druckergebnisse mit violetten bis blaugrünen Aufzeichnungen wurden beim Einsatz entsprechender Farbstoffgeberelemente erhalten, welche die Produkte der Beispiele 2 bis 29 enthielten.

### Beispiel 31 (thermisch entwickelbares Aufzeichnungselement)

Eine 0,5 gew.-%ige Lösung des Farbstoffs erhalten gemäß Beispiel 4 in Chloroform, die intensiv blau gefärbt war, wurde auf ein Blatt Papier aufgetragen. Nach der Verdampfung des Lösungsmittels verschwand die blaue Farbe und das beschichtete Papier war nur noch schwach beige gefärbt. Beim Beschreiben des Papiers mit einem erhitzten Glasstab bildete sich ein intensiver und beständiger violett-blauer Schriftzug aus.

Analoge Ergebnisse wurden erhalten, wenn statt des Blatt Papiers eine Folie aus Polyethylenterephthalat eingesetzt wurde und statt des erhitzten Glasstabs ein Thermodrucker verwendet wurde.

Analoge Ergebnisse wurden weiterhin erhalten, wenn das Aufzeichnungselement mit Lösungen der Farbstoffe gemäß den Beispielen 1 bis 3 und 5 bis 29 hergestellt wurde.

### Beispiel 32 (photochemisch entwickelbares Aufzeichnungselement)

Eine 0,5 gew.-%ige Lösung des gemäß Beispiei 9 erhaltenen Farbstoffs in Tetrahydrofuran, die intensiv blau gefärbt ist, wurde auf eine Folie aus Polyethylenterephthalat aufgetragen. Nach der Verdampfung des Lösungsmittels war die blaue Farbe verschwunden und die Beschichtung nur noch schwach beige gefärbt. Bei der Belichtung der Folie mit einer Blitzlichtlampe färbte sich die Beschichtung intensiv türkisfarben. Diese Farbe blieb über mehrere Tage unverändert bestehen.

Analoge Ergebnisse wurden beim entsprechenden Einsatz der Farbstoffe gemäß den Beispielen 1 bis 8 und 10 bis 29 erhalten.

Analoge Ergebnisse wurden weiterhin erhalten, wenn statt mit einer Blitzlichtlampe mit einem Farbstofflaser (Wellenlänge 460 µm, Cumarin 47 der Fa. Lambda Physik) belichtet wurde.

### Beispiel 33 (nichtlineares optisches Element)

In einer Apparatur ähnlich der von D. Lupo et al. in Adv. Mater. 3, 54 (1991) beschriebenen wurde der gemäß Beispiel 4 erhaltene Farbstoff in Form von kristallinem Pulver mit Laserlicht der Wellenlänge 1064 nm bestrahlt. Dabei wurde die Emission von Licht der halben Wellenlänge (= 532 nm) beobachtet. Wurde die Bestrahlung mit Laserlicht der Wellenlänge 820 nm vorgenommen, so wurde Licht mit einer Wellenlänge von 410 nm emitiert.

Analoge Ergebnisse wurden erhalten, wenn der Farbstoff erhalten gemäß Beispiel 23 mit Laserlicht bestrahlt wurde, nachdem er in Form eines Films von einer Wasseroberfläche auf eine Glasplatte aufgebracht worden war.

Analoge Ergebnisse wurden ebenfalls erhalten, wenn Farbstoffe gemäß den Beispielen 1 bis 3, 5 bis 22, 24 und 29 eingesetzt wurden.

### Beispiel 34 (Photorezeptorelement)

Ein Photorezeptorelement wurde entsprechend Beispiel 11 der US-PS 4 471 041 hergestellt. Statt des dort verwendeten Farbstoffs wurde der gemäß Beispiel 2 erhaltene Farbstoff eingesetzt.

Es wurde ein Photorezeptorelement erhalten, das auf einem Aluminiumträger eine photoleitende Schicht des gemäß Beispiel 2 erhaltenen Farbstoffs in einem Polyesterbinder (1 µm dick) enthielt und darüber eine Ladungstransportschicht enthaltend N,N'-Diphenyl-N,N'-(3-methylphenyl)-1,1'-biphenyl-4,4'-diamin in einem Polycarbonatbinder.

Dieses Element wurde mit einer Corona-Entladung auf -760 V aufgeladen. Die Belichtung mit einer Intensität von 150 mJ/m² bei einer Wellenlänge von 600 nm führte zu einer Entladung von 60 %.

Analoge Ergebnisse wurden erhalten, wenn gemäß den Beispielen 1 und 3 bis 29 erhaltene Farbstoffe eingesetzt wurden.

### Beispiel 35 (Polyesterfärbung)

0,1 g des Farbstoffs erhalten gemäß Beispiel 8 wurde in Dimethylformamid gelöst und in 300 ml Wasser, dem als Carrier 0,6 g Avolan® JS, 0,3 g Natriumdihydrogenphosphat und Essigsäure bis zum Erreichen eines pH-Wertes von 4,5 zugesetzt worden war, gegeben. 10 g eines Polyestergewebes aus Trevira® 2000 wurden zugefügt und bei 130°C gefärbt. Das Gewebe wies danach einen graublauen Farbton auf.

Analoge Ergebnisse wurden erhalten, wenn die Farbstoffe, erhalten gemäß den Beispielen 1 bis 7 und 9 bis 29, eingesetzt wurden.

### Beispiel 36 (Kunststoffärbung)

0,1 g des Farbstoffs erhalten gemäß Beispiel 2 und 2 g Titandioxid wurden in 100 g Polystyrol eingearbeitet und spritzgegossen. Man erhielt einen intensiv blau eingefärbten Kunststoffspritzling.

Analoge Ergebnisse wurden erhalten, wenn die Farbstoffe, erhalten gemäß den Beispielen 1 und 3 bis 29, eingesetzt wurden.

## Patentansprüche

1. Azinneutromethine der Formel (I) 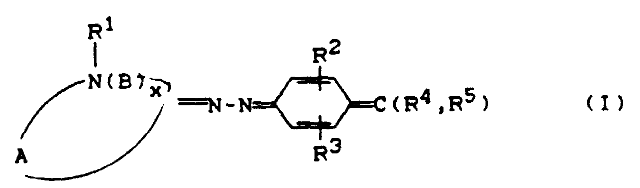 bei denen
R¹ für C₁- bis C₂₂-Alkyl, C₄- bis C₁₀-Cycloalkyl, C₇- bis C₁₄-Aralkyl, C₆- bis C₁₀-Aryl oder einen gegebenenfalls über C₁- bis C₂-Alkylen gebundenen, 1 bis 4 Stickstoff-, Sauerstoff- und/oder Schwefelatome und 4 bis 12 C-Atome enthaltenden heterocyclischen Rest steht, wobei alle diese Reste gegebenenfalls substituiert sein können,
R² und R³ unabhängig voneinander für Wasserstoff, Halogen, Hydroxy, Cyano, C₁- bis C₈-Alkyl, C₁- bis C₈-Alkoxy, C₄- bis C₁₀-Cycloalkoxy, C₇- bis C₁₄-Aralkyloxy oder C₁- bis C₂₀-Acylamino stehen,
R⁴ und R⁵ unabhängig voneinander für Cyano, C₁- bis C₈-Alkoxycarbonyl, C₄- bis C₇-Cycloalkoxycarbonyl, C₆- bis C₁₀-Aryloxycarbonyl, Aminocarbonyl, Mono-C₁- bis C₈-alkylaminocarbonyl, Di-C₁- bis C₈-alkylaminocarbonyl, C₆- bis C₁₀-Arylaminocarbonyl, mit N-, S- undloder O-haltigen 5 bis 7 Atome enthaltenden Heterocyclen substituiertes Aminocarbonyl oder durch Cyano und/oder Nitro substituiertes Phenyl stehen oder
R⁴ und R⁵ gemeinsam mit dem dazwischenliegenden C-Atom für einen gegebenenfalls bis zu 2 Stickstoff- und/oder Sauerstoffatome enthaltenden, gegebenenfalls durch Cyano, Halogen, C₁- bis C₄-Alkyl oder C₆- bis C₁₀- Aryl substituierten 5 oder 6 Atome enthaltenden Ring stehen und
A den Ring, in dem es sich befindet, ergänzt zu einem
gegebenenfalls benz- oder naphthanellierten, gegebenenfalls ein weiteres Stickstoff-, Sauerstoff- oder Schwefelatom enthaltenden, gegebenenfalls durch Cyano, Nitro, C₁- bis C₄-Alkyl, C₇-bis C₁₂-Aralkyl, C₆- bis C₁₀-Aryl, C₁-bis C₄-Alkoxy, gegebenenfalls über C₁-bis C₂-Alkylen gebundenen bis zu 2 N-, S- und/oder O-Atome enthaltenden 5- bis 6-gliedrigen Ring, C₁- bis C₄-Alkylthio, C₆-bis C₁₀-Arylthio, Amino, Mono- oder Di-C₁- bis C₈-alkylamino, C₄- bis C₈-Cycloalkyl-C₁- bis C₄-alkylamino, C₇- bis C₁₂-Aryl, C₁- bis C₄-alkylamino, Mono- oder Di-C₆-bis C₁₀-arylamino, Pyrrolidino, Piperidino, Morpholino oder C₁- bis C₆-Acylamino substituierten
5- bis 7-gliedrigen aromatischen oder quasiaromatischen Ring,
B für CR^{B} = CH mit R^{B} = Wasserstoff oder C₁ bis C₆-Alkyl und
x für Null oder 1 stehen.

2. Azinneutromethine nach Anspruch 1, dadurch gekennzeichnet, daß sich in der Formel (I) die Reste
R² in ortho-Stellung zur 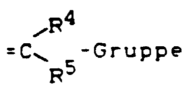 und
R³ in ortho-Stellung zur =N-N= -Gruppe und in para-Stellung zu R² befinden und
R¹ für gegebenenfalls verzweigtes und/oder durch insgesamt bis zu drei Substituenten aus der Gruppe C₁- bis C₄-Alkoxy, Fluor, Chlor, Brom, Hydroxy, Cyano und Aminocarbonyl substituiertes C₁- bis C₂₂-Alkyl, Cyclopentyl, Cyclohexyl, Benzyl, Phenethyl, gegebenenfalls durch insgesamt bis zu 2 Substituenten aus der Gruppe C₁- bis C₄-Alkyl, C₁- bis C₄-Alkoxy, Chlor und Cyano substituiertes Phenyl oder Pyridylethyl steht,
R² für C₁- bis C₄-Alkyl, C₁- bis C₄-Alkoxy, Chlor, Brom, Hydroxy oder Cyano steht,
R³ für Wasserstoff, C₁- bis C₄-Alkyl, C₁- bis C₄-Alkoxy, Chlor, Brom, Hydroxy, Cyano, gegebenenfalls durch mit bis zu neun Fluoratomen substituiertes C₁- bis C₂₀-Alkanoylamino oder gegebenenfalls durch insgesamt bis zu zwei Substituenten aus der Gruppe Methyl, Methoxy und Chlor substituiertes Benzoylamino steht,
R⁴ für Cyano, C₁- bis C₄-Alkoxycarbonyl, Cyclopentoxycarbonyl, Cyclohexoxycarbonyl, Phenoxycarbonyl, Aminocarbonyl, Mono- oder Di-C₁- bis C₄-alkylaminocarbonyl, Anilinocarbonyl oder Nitrophenyl steht,
R⁵ für Cyano steht,
A den Ring, in dem es sich befindet, ergänzt zu einem Pyrazol-, Imidazol-, Triazol-, Thiazol-, Thiadiazol-, Benzimidazol-, Benzthiazol-, Pyridin-, Pyrazin-, Pyrimidin- oder Chinolinring,
wobei diese Ringe gegebenenfalls durch insgesamt bis zu zwei Substituenten aus der Gruppe Chlor, Cyano, Phenyl, C₁- bis C₄-Alkyl, C₁-bis C₄-Alkoxy, C₁- bis C₄-Alkylthio, Phenylthio, Di-C₁- bis C₄-alkylamino, Cyclohexyl-C₁-bis C₄-alkylamino, Benzyl-C₁- bis C₄-alkylamino, Phenylamino, Phenyl-C₁- bis C₄-alkylamino, Pyrrolidino, Piperidino oder Morpholino substituiert sein können
wobei die Substituenten C₁- bis C₄-Alkoxy, C₁- bis C₄-Alkylthio, Di-C₁- bis C₄-alkylamino und Cyclohexyl-C₁- bis C₄-alkylamino ihrerseits gegebenenfalls insgesamt bis zu zwei Substituenten aus der Gruppe Chlor, Methoxy, Ethoxy, Hydroxy und Cyano und die Substituenten Phenylamino und Phenyl-C₁- bis C₄-alkylamino ihrerseits gegebenenfalls insgesamt bis zu zwei Substituenten aus der Gruppe Methyl, Methoxy, Cyano und Chlor enthalten können und
B für CH = CH und
x für Null oder 1 stehen.

3. Azinneutromethine nach Ansprüchen 1 und 2, dadurch gekennzeichnet, daß sich in der Formel (I) die Reste
R² in ortho-Stellung zur 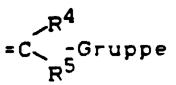 und
R³ in ortho-Stellung zur =N-N= -Gruppe und in para-Stellung zu R² befinden und
R¹ für gegebenenfalls verzweigtes und/oder durch eine Chlor-, Cyano- oder Methoxygruppe substituiertes C₁- bis C₁₆-Alkyl oder für Benzyl steht,
R² für Methyl, Ethyl, Methoxy, Ethoxy oder Chlor steht,
R³ für Wasserstoff, Methyl, Ethyl, Methoxy, Ethoxy, Chlor, Cyano, Formylamino, Acetylamino, Propionylamino, Butyroylamino, Octanoylamino, Stearoylamino, Trifluoracetylamino, Nonafluorbutyroxyamino, Benzoylamino, Methylbenzoylamino oder Chlorbenzoylamino steht,
R⁴ für Cyano, Methoxycarbonyl, Ethoxycarbonyl, Amidocarbonyl oder 4-Nitrophenyl steht,
R⁵ für Cyano steht,
A den Ring, in dem es sich befindet, ergänzt zu einem der Ringe (II) bis (VI) 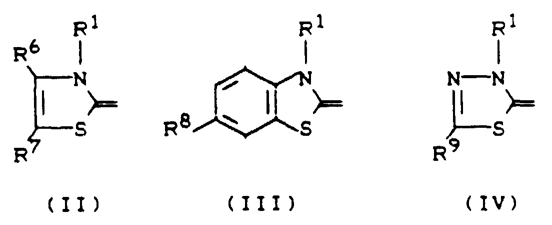 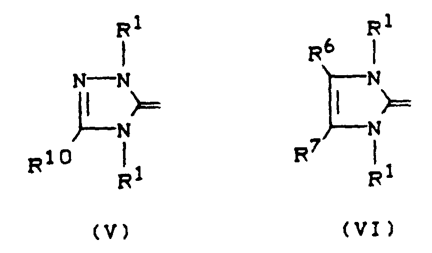 in denen
R¹ die oben angegebene Bedeutung hat,
R⁶ für Wasserstoff, Methyl, Ethyl, n-Propyl, i-Propyl, tert.-Butyl oder Phenyl und
R⁷ für Wasserstoff, Methyl, Ethyl, Chlor oder Brom oder
R⁶ und R⁷ gemeinsam für -(CH₂)₄- stehen,
R⁸ für Wasserstoff, Methyl, Methoxy oder Chlor und
R⁹ für Wasserstoff, Methyl, Ethyl, Phenyl, Methylthio, Phenylthio, Anilino, 4-Methylanilino, 4-Methoxyanilino, N-Methylanilino, Dimethylamino, Diethylamino, Dipropylamino, Dibutylamino, Dihydroxyethylamino, Dihydroxypropylamino, Hydroxyethylcyclohexylamino, Pyrrolidino, Piperidino oder Morpholino stehen,
R¹⁰ für Wasserstoff, Methyl, Ethyl, Phenyl oder Hydroxypropyl und
x für Null steht.

4. Azinneutromethine nach Anspruch 3, dadurch gekennzeichnet, daß
R² für Methoxy und
R⁴ für Cyano steht.

5. Verfahren zur Herstellung der Azinneutromethine des Anspruchs 1, dadurch gekennzeichnet, daß man eine Verbindung der Formel (VII) 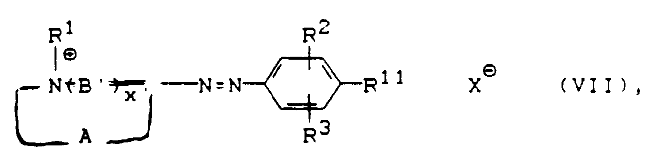 in der
R¹, R², R³ und A die bei der Formel (I) angegebene Bedeutung haben,
B' für CR^{B'}-CH mit R^{B'} = Wasserstoff oder C₁- bis C₆-Alkyl steht,
x' für Null oder 1 steht,
R¹¹ für Halogen, Alkoxy, Phenoxy, Monoalkylamin Dialkylamino, N-Cycloalkyl-N-alkylamino oder N-Aralkyl-N-alkylamino steht und
X^{⊖} für ein Anion steht,
mit einer methylenaktiven Verbindung der Formel (VIII) 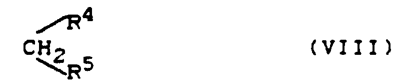 in der
R⁴ und R⁵ die bei der Formel (I) angegebene Bedeutung haben,
umsetzt.

6. Verfahren nach Anspruch 5, dadurch gekennzeichnet, daß man es bei Temperaturen zwischen 0°C und dem Siedepunkt des jeweiligen Reaktionsgemisches, in Gegenwart eines Lösungsmittels und in Gegenwart einer Base durchführt.

7. Verwendung von Azinneutromethinen des Anspruchs 1 in thermischen und fotochromen Aufzeichnungsmaterialien, als nicht lineare optische Materialien und als Fotoleiter in der Elektrofotografie.

8. Verwendung von Azinneutromethinen des Anspruchs 1 zum Färben von Fasern und Geweben aus Polyester und von Kunststoffen.

## Claims

1. Azinoneutromethines of the formula (I) 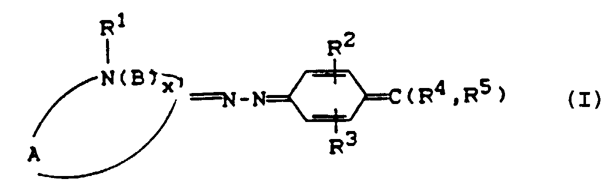 in which
R¹ represents C₁- to C₂₂-alkyl, C₄- to C₁₀-cycloalkyl, C₇- to C₁₄-aralkyl, C₆- to C₁₀-aryl, or a heterocyclic radical which is optionally bonded via C₁- to C₂-alkylene and contains 1 to 4 nitrogen, oxygen and/or sulphur atoms and 4 to 12 C atoms, it being possible for all these radicals optionally to be substituted,
R² and R³ independently of one another represent hydrogen, halogen, hydroxyl, cyano, C₁- to C₈-alkyl, C₁- to C₈-alkoxy, C₄- to C₁₀-cycloalkoxy, C₇- to C₁₄-aralkyloxy or C₁- to C₂₀-acylamino,
R⁴ and R⁵ independently of one another represent cyano, C₁- to C₈-alkoxycarbonyl, C₄- to C₇-cycloalkoxycarbonyl, C₆- to C₁₀-aryloxycarbonyl, aminocarbonyl, mono-C₁- to C₈-alkylaminocarbonyl, di-C₁- to C₈-alkylaminocarbonyl, C₆- to C₁₀-arylaminocarbonyl, aminocarbonyl which is substituted by heterocyclic radicals which contain 5 to 7 atoms and contain N, S and/or 0, or phenyl which is substituted by cyano and/or nitro, or
R⁴ and R⁵, together with the C atom in between, represent a ring which contains 5 or 6 atoms, optionally contains up to 2 nitrogen and/or oxygen atoms and is optionally substituted by cyano, halogen, C₁- to C₄-alkyl or C₆- to C₁₀-aryl, and
A complements the ring in which it is located to form a
5- to 7-membered aromatic or quasiaromatic ring which is
optionally benzo- or naphtho-fused, optionally contains a further nitrogen, oxygen or sulphur atom, and is optionally substituted by cyano, nitro, C₁- to C₄- alkyl, C₇- to C₁₂-aralkyl, C₆- to C₁₀-aryl, C₁- to C₄- alkoxy, a 5- to 6-membered ring which is optionally bonded via C₁- to C₂-alkylene and contains up to 2 N, S and/or O atoms, C₁- to C₄-alkylthio, C₆- to C₁₀-arylthio, amino, mono- or di-C₁- to C₈-alkylamino, C₄- to C₈-cycloalkyl-C₁- to C₄-alkylamino, C₇- to C₁₂-aryl-C₁- to C₄-alkylamino, mono- or di-C₆- to C₁₀-arylamino, pyrrolidino, piperidino, morpholino or C₁- to C₆-acylamino,
B represents CR^{B}=CH, where R^{B} = hydrogen or C₁- to C₆-alkyl, and
x represents zero or 1.

2. Azinoneutromethines according to Claim 1, characterised in that, in formula (I), the radical
R² is in the ortho-position relative to the 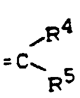 group and
R³ is in the ortho-position relative to the =N-N= group and in the para-position relative to R², and
R¹ represents C₁- to C₂₂- alkyl which is optionally branched and/or substituted by a total of up to three substituents from the group comprising C₁- to C₄-alkoxy, fluorine, chlorine, bromine, hydroxyl, cyano and aminocarbonyl, cyclopentyl, cyclohexyl, benzyl, phenethyl, phenyl which is optionally substituted by a total of up to 2 substituents from the group comprising C₁- to C₄-alkyl, C₁- to C₄-alkoxy, chlorine and cyano, or pyridylethyl,
R² represents C₁- to C₄-alkyl, C₁- to C₄-alkoxy, chlorine, bromine, hydroxyl or cyano,
R³ represents hydrogen, C₁- to C₄-alkyl, C₁- to C₄-alkoxy, chlorine, bromine, hydroxyl, cyano, C₁-to C₂₀-alkanoylamino which is optionally substituted by up to nine fluorine atoms, or benzoylamino which is optionally substituted by a total of up to two substituents from the group comprising methyl, methoxy and chlorine,
R⁴ represents cyano, C₁- to C₄-alkoxycarbonyl, cyclopentoxycarbonyl, cyclohexoxycarbonyl, phenoxycarbonyl, aminocarbonyl, mono- or di-C₁-to C₄-alkylaminocarbonyl, anilinocarbonyl or nitrophenyl,
R⁵ represents cyano,
A complements the ring in which it is located to form a pyrazole, imidazole, triazole, thiazole, thiadiazole, benzimidazole, benzothiazole, pyridine, pyrazine, pyrimidine or quinoline ring,
it being possible for these rings optionally to be substituted by a total of up to two substituents from the group comprising chlorine, cyano, phenyl, C₁- to C₄-alkyl, C₁- to C₄-alkoxy, C₁- to C₄-alkylthio, phenylthio, di-C₁- to C₄-alkylamino, cyclohexyl-C₁- to C₄-alkylamino, benzyl-C₁- to C₄-alkylamino, phenylamino, phenyl-C₁- to C₄-alkylamino, pyrrolidino, piperidino or morpholino,
it being possible for the substituents C₁- to C₄-alkoxy, C₁- to C₄-alkylthio, di-C₁- to C₄-alkylamino and cyclohexyl-C₁- to C₄-alkylamino in turn optionally to contain a total of up to two substituents from the group comprising chlorine, methoxy, ethoxy, hydroxyl and cyano, and for the substituents phenylamino and phenyl-C₁- to C₄-alkylamino in turn optionally to contain a total of up to two substituents from the group comprising methyl, methoxy, cyano and chlorine, and
B represents CH=CH and
x represents zero or 1.

3. Azinoneutromethines according to Claims 1 and 2, characterised in that, in formula (I), the radical
R² is in the ortho-position relative to the 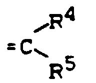 group and
R³ is in the ortho-position relative to the =N-N= group and in the para-position relative to R², and
R¹ represents C₁- to C₁₆-alkyl which is optionally branched and/or substituted by a chlorine, cyano or methoxy group, or represents benzyl,
R² represents methyl, ethyl, methoxy, ethoxy or chlorine,
R³ represents hydrogen, methyl, ethyl, methoxy, ethoxy, chlorine, cyano, formylamino, acetylamino, propionylamino, butyroylamino, octanoylamino, stearoylamino, trifluoroacetylamino, nonafluorobutyroylamino, benzoylamino, methylbenzoylamino or chlorobenzoylamino,
R⁴ represents cyano, methoxycarbonyl, ethoxycarbonyl, amidocarbonyl or 4-nitrophenyl,
R⁵ represents cyano,
A complements the ring in which it is located to form one of the rings (II) to (VI) 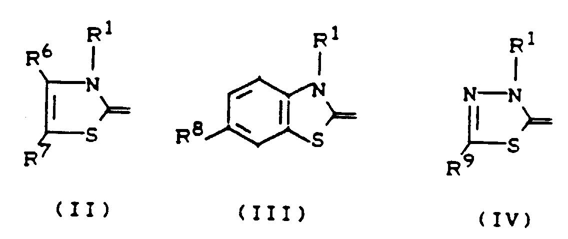 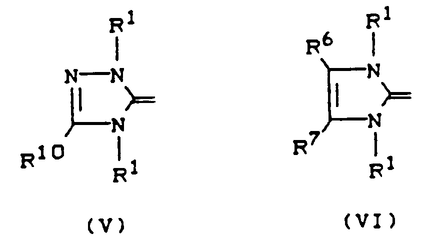 in which
R¹ has the abovementioned meaning,
R⁶ represents hydrogen, methyl, ethyl, n-propyl, i-propyl, tert-butyl or phenyl and
R⁷ represents hydrogen, methyl, ethyl, chlorine or bromine, or
R⁶ and R⁷ together represent -(CH₂)₄-,
R⁸ represents hydrogen, methyl, methoxy or chlorine,
R⁹ represents hydrogen, methyl, ethyl, phenyl, methylthio, phenylthio, anilino, 4-methylanilino, 4-methoxyanilino, N-methylanilino, dimethylamino, diethylamino, dipropylamino, dibutylamino, dihydroxyethylamino, dihydroxypropylamino, hydroxyethylcyclohexylamino, pyrrolidino, piperidino or morpholino and
R¹⁰ represents hydrogen, methyl, ethyl, phenyl or hydroxypropyl, and
x represents zero.

4. Azinoneutromethines according to Claim 3, characterised in that
R² represents methoxy and
R⁴ represents cyano.

5. Process for the preparation of the azinoneutromethines of Claim 1, characterised in that a compound of the formula (VII) 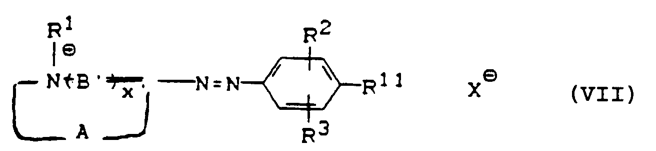 in which
R¹, R², R³ and A have the meaning given in the case of formula (I),
B' represents CR^{B'}-CH, where R^{B'} = hydrogen or C₁- to C₆-alkyl,
x' represents zero or 1,
R¹¹ represents halogen, alkoxy, phenoxy, monoalkyl amino , dialkylamino, N-cycloalkyl-N-alkylamino or N-aralkyl-N-alkylamino and
X^{⊖} represents an anion,
is reacted with a methylene-active compound of the formula (VIII) 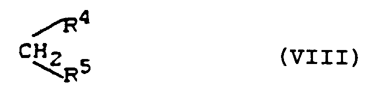 in which
R⁴ and R⁵ have the meaning given in the case of formula (I).

6. Process according to Claim 5, characterised in that it is carried out at temperatures between 0°C and the boiling point of the particular reaction mixture, in the presence of a solvent and in the presence of a base.

7. Use of azinoneutromethines of Claim 1 in thermal and photochromic recording materials, as nonlinear optical materials and as photoconductors in electrophotography.

8. Use of azinoneutromethines of Claim 1 for dyeing fibres and woven fabrics of polyester and plastics.

## Revendications

1. Neutrométhines aziniques de formule (I) 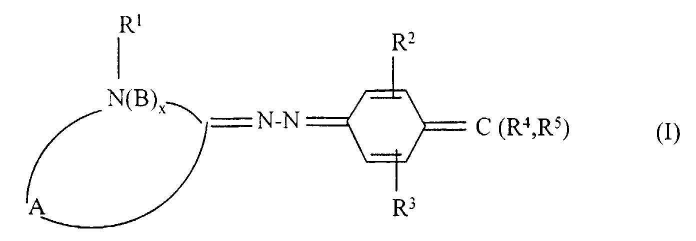 dans laquelle
R¹ représente un groupe alkyle en C₁-C₂₂, cycloalkyle en C₄-C₁₀, aralkyle en C₇-C₁₄, aryle en C₆-C₁₀ ou un radical hétérocyclique éventuellement relié par l'intermédiaire d'un groupe alkylène en C₁-C₂, et contenant 1 à 4 atomes d'azote, d'oxygène et/ou de soufre et 4 à 12 atomes de carbone, tous ces radicaux pouvant le cas échéant être substitués,
R² et R³ représentent chacun, indépendamment l'un de l'autre, l'hydrogène, un halogène, un groupe hydroxy, cyano, alkyle en C₁-C₈, alcoxy en C₁-C₈, cycloalcoxy en C₄-C₁₀, aralkyloxy en C₇-C₁₄ ou acylamino en C₁-C₂₀,
R⁴ et R⁵ représentent chacun, indépendamment l'un de l'autre, un groupe cyano, (alcoxy en C₁-C₈)carbonyle, (cycloalcoxy en C₄-C₇)carbonyle, (aryloxy en C₆-C₁₀)carbonyle, aminocarbonyle, mono-(alkyle en C₁-C₈)aminocarbonyle, di(alkyle en C₁-C₈)aminocarbonyle, (arylamino en C₆-C₁₀)carbonyle, aminocarbonyle substitué par des hétérocycles de 5 à 7 atomes contenant N, S et/ou O, ou un groupre phényle portant des substituants cyano et/ou nitro, ou bien
R⁴ et R⁵ représentent, ensemble et avec l'atome de carbone auquel ils sont reliés, un cycle de 5 ou 6 atomes contenant éventuellement jusqu'à 2 atomes d'azote et/ou d'oxygène et le cas échéant substitué par des groupes cyano, des halogènes, des groupes alkyle en C₁-C₄ ou aryle en C₆-C₁₀, et
A complète le cycle dans lequel il se trouve en un cycle aromatique ou quasi-aromatique de 5 à 7 chaînons éventuellement condensé avec un cycle benzénique ou naphtalénique, contenant le cas échéant un autre atome d'azote, d'oxygène ou de soufre, le cas échéant substitué par des groupes cyano, nitro, alkyle en C₁-C₄, aralkyle en C₇-C₁₂, aryle en C₆-C₁₀, alcoxy en C₁-C₄, un cycle de 5 à 6 chaînons reliés éventuellement par l'intermédiaire d'un groupe alkylène en C₁-C₂, et contenant jusqu'à 2 atomes d'azote, de soufre et/ou d'oxygène, des groupes alkylthio en C₁-C₄, arylthio en C₆-C₁₀, amino, mono- ou di-(alkyle en C₁-C₈)amino, (cycloalkyle en C₄-C₈)-alkylamino en C₁-C₄, (aryle en C₇-C₁₂)-alkylamino en C₁-C₄, mono- ou di-(aryle en C₆-C₁₀)amino, pyrrolidino, pipéridino, morpholino ou acylamino en C₁-C₆,
B représente CR^{B} = CH avec R^{B} = hydrogène ou alkyle en C₁-C₆ et
x est égal à 0 ou 1.

2. Neutrométhines aziniques selon revendication 1 caractérisées en ce que, dans la formule (I),
R² est en position ortho du groupe 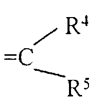 et
R³ est en position ortho du groupe =N-N= et en position para de R² et
R¹ représente un groupe alkyle en C₁-C₂₂ éventuellement ramifié et/ou portant au total jusqu'à 3 substituants choisis parmi les groupes alcoxy en C₁-C₄, le fluor, le chlore, le brome, les groupes hydroxy, cyano et aminocarbonyle, un groupe cyclopentyle, cyclohexyle, benzyle, phénéthyle, un groupe phényle portant éventuellement jusqu'à 2 substituants au total choisis parmi les groupes alkyle en C₁-C₄, alcoxy en C₁-C₄, le chlore et les groupes cyano, ou un groupe pyridyléthyle,
R² représente un groupe alkyle en C₁-C₄, alcoxy en C₁-C₄, le chlore, le brome, un groupe hydroxy ou cyano,
R³ représente l'hydrogène, un groupe alkyle en C₁-C₄, alcoxy en C₁-C₄, le chlore, le brome, un groupe hydroxy, cyano, un groupe alcanoylamino en C₁-C₂₀ portant éventuellement jusqu'à 9 substituants fluoro ou un groupe benzoylamino portant éventuellement jusqu'à 2 substituants au total choisis parmi les groupes méthyle, méthoxy et le chlore,
R⁴ représente un groupe cyano, (alcoxy en C₁-C₄)carbonyle, cyclopentoxycarbonyle, cyclohexoxycarbonyle, phénoxycarbonyle, aminocarbonyle, mono- ou di-(alkyle en C₁-C₄)aminocarbonyle, anilinocarbonyle ou nitrophényle,
R⁵ représente un groupe cyano,
A complète le cycle dans lequel il se trouve en un cycle pyrazole, imidazole, triazole, thiazole, thiadiazole, benzimidazole, benzothiazole, pyridine, pyrazine, pyrimidine ou quinoléine, ces cycles pouvant le cas échéant porter au total jusqu'à 2 substituants choisis parmi le chlore, les groupes cyano, phényle, alkyle en C₁-C₄, alcoxy en C₁-C₄, alkylthio en C₁-C₄, phénylthio, di(alkyle en C₁-C₄)amino, cyclohexyl-alkylamino en C₁-C₄, benzyl-alkylamino en C₁-C₄, phénylamino, phényl-alkylamino en C₁-C₄, pyrrolidino, pipéridino ou morpholino,
les substituants alcoxy en C₁-C₄, alkylthio en C₁-C₄, di(alkyle en C₁-C₄)amino et cyclohexyl-alkylamino en C₁-C₄, pouvant eux-mêmes le cas échéant porter jusqu'à 2 substituants au total choisis parmi le chlore, les groupes méthoxy, éthoxy, hydroxy et cyano, et les substituants phénylamino et phényl-alkylamino en C₁-C₄ pouvant eux-mêmes le cas échéant porter au total jusqu'à 2 substituants choisis parmi les groupes méthyle, méthoxy, cyano et le chlore,
B représente CH = CH et
x est égal à 0 ou 1.

3. Neutrométhines aziniques selon les revendications 1 et 2 caractérisées en ce que, dans la formule (I),
R² est en position ortho du groupe 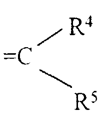 et
R³ est en position ortho du groupe =N-N= et en position para de R² et
R¹ représente un groupe alkyle en C₁-C₁₆ éventuellement ramifié et/ou substitué par un atome de chlore, un groupe cyano ou méthoxy, ou un groupe benzyle,
R² représente un groupe méthyle, éthyle, méthoxy, éthoxy ou le chlore, R³ représente l'hydrogène, un groupe méthyle, éthyle, méthoxy, éthoxy, le chlore, un groupe cyano, formylamino, acétylamino, propionylamino, butyrylamino, octanoylamino, stéaroylamino, trifluoracétylamino, nonafluorobutyrylamino, benzoylamino, méthylbenzoylamino ou chlorobenzoylamino,
R⁴ représente un groupe cyano, méthoxycarbonyle, éthoxycarbonyle, amidocarbonyle ou 4-nitrophényle,
R⁵ représente un groupe cyano,
A complète le cycle dans lequel il se trouve en l'un des cycles (II) à (VI) 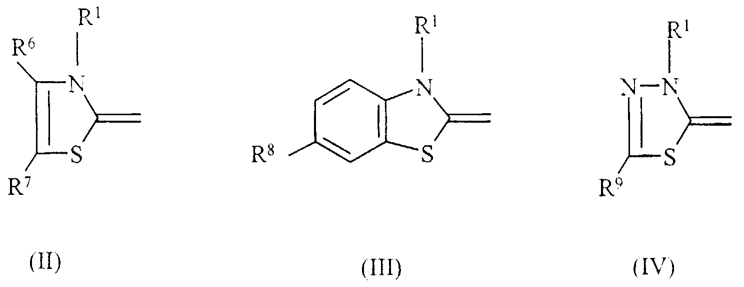 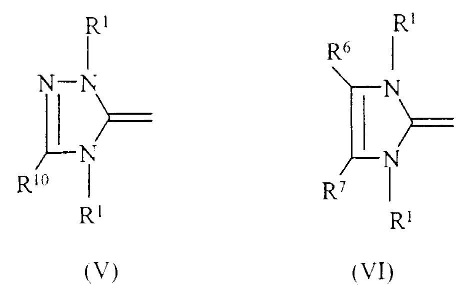 dans lesquels
R¹ a les significations indiquées ci-dessus,
R⁶ représente l'hydrogène, un groupe méthyle, éthyle, n-propyle, isopropyle, tert.-butyle ou phényle,
R⁷ représente l'hydrogène, un groupe méthyle, éthyle, le chlore ou le brome, ou bien
R⁶ et R⁷ représentent ensemble -(CH₂)-₄,
R⁸ représente l'hydrogène, un groupe méthyle, méthoxy ou le chlore et
R⁹ représente l'hydrogène, un groupe méthyle, éthyle, phényle, méthylthio, phénylthio, anilino, 4-méthylanilino, 4-méthoxyanilino, N-méthylanilino, diméthylamino, diéthylamino, dipropylamino, dibutylamino, dihydroxyéthylamino, dihydroxypropylamino, hydroxyéthylcyclohexylamino, pyrrolidino, pipéridino ou morpholino,
R¹⁰ représente l'hydrogène, un groupe méthyle, éthyle, phényle ou hydroxypropyle et
x est égal à 0.

4. Neutrométhines aziniques selon la revendication 3, caractérisées en ce que
R² représente un groupe méthoxy et
R⁴ un groupe cyano.

5. Procédé de préparation des neutrométhines aziniques de la revendication 1, caractérisé en ce que l'on fait réagir un composé de formule (VII) 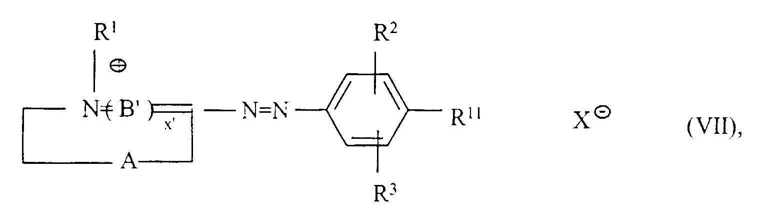 dans laquelle
R¹, R², R³ et A ont les significations indiquées en référence à la formule (I),
B' représente CR^{B'}-CH avec R^{B'} = hydrogène ou alkyle en C₁-C₆,
x' est égal à 0 ou 1,
R¹¹ représente un halogène, un groupe alcoxy, phénoxy, monoalkylamino, dialkylamino, N-cycloalkyl-N-alkylamino ou N-aralkyl-N-alkylamino et
X^{⊖} représente un anion,
avec un composé à groupe méthylène actif de formule (VIII) 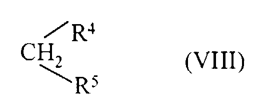 dans laquelle
R⁴ et R⁵ ont les significations indiquées en référence à la formule (I).

6. Procédé selon la revendication 5 caractérisé en ce qu'il est mis en oeuvre à des températures comprises entre 0°C et le point d'ébullition du mélange de réaction en présence d'un solvant et en présence d'une base.

7. Utilisation des neutrométhines aziniques de la revendication 1 dans des matériaux d'enregistrement thermiques et photochromes, en tant que matières optiques non linéaires et en tant que photoconducteurs en électrophotographie.

8. Utilisation des neutrométhines aziniques de la revendication 1 pour la teinture de fibres et de tissus en polyester et pour la coloration de résines synthétiques.
